(19) Europäisches Patentamt / European Patent Office / Office européen des brevets

(11) EP 1 326 908 B1

(12) # FASCICULE DE BREVET EUROPEEN

(45) Date de publication et mention de la délivrance du brevet:
**28.05.2008 Bulletin 2008/22**

(21) Numéro de dépôt: **01976410.9**

(22) Date de dépôt: **12.10.2001**

(51) Int Cl.:
***C08G 18/08*** *(2006.01)* ***C08G 18/48*** *(2006.01)*
***C08G 18/66*** *(2006.01)* ***A61K 8/87*** *(2006.01)*
***A61Q 1/02*** *(2006.01)* ***A61Q 5/06*** *(2006.01)*

(86) Numéro de dépôt international:
**PCT/FR2001/003157**

(87) Numéro de publication internationale:
**WO 2002/032978 (25.04.2002 Gazette 2002/17)**

(54) **POLYURETHANNES CATIONIQUES A CARACTERE ELASTIQUE**

KATIONISCHE POLYURETHANE MIT ELASTISCHEM CHARAKTER

CATIONIC POLYURETHANES WITH ELASTIC CHARACTER

(84) Etats contractants désignés:
**AT BE CH CY DE DK ES FI FR GB GR IE IT LI LU MC NL PT SE TR**

(30) Priorité: **17.10.2000 FR 0013264**

(43) Date de publication de la demande:
**16.07.2003 Bulletin 2003/29**

(73) Titulaire: **L'ORÉAL**
**75008 Paris (FR)**

(72) Inventeur: **MOUGIN, Nathalie**
**F-75011 Paris (FR)**

(74) Mandataire: **Casalonga, Axel et al**
**Bureau Casalonga & Josse**
**Bayerstrasse 71/73**
**80335 München (DE)**

(56) Documents cités:
**DE-A- 4 241 118**
**GB-A- 2 039 930**
**US-A- 3 388 087**
**US-A- 4 110 286**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne de nouveaux polyuréthannes cationiques à caractère élastique ainsi que leur utilisation dans des compositions cosmétiques.

La formation de dépôts et de films à propriétés élastiques fait depuis toujours l'objet d'importantes recherches en cosmétique. En effet, la plupart des zones du corps humain susceptibles de recevoir des dépôts cosmétiques, telles que la peau, les lèvres, les cheveux, les cils et les ongles sont soumises à des déformations et contraintes mécaniques importantes. Les films et dépôts cosmétiques doivent pouvoir résister à ces contraintes et suivre ces déformations sans se casser.

L'utilisation de polyuréthannes en cosmétique est connue depuis longtemps et est décrite par exemple dans les brevets WO94/13724 (DE 42 41 118) et EP 0 619 111.

Les polyuréthannes divulgués dans ces documents présentent cependant des températures de transition vitreuse (Tg) supérieures à la température ambiante (20 °C), c'est-à-dire à la température ambiante ils sont à l'état vitreux et forment des films cassants inacceptables pour une application cosmétique.

Il existe certes des polymères physiologiquement acceptables présentant des températures de transition vitreuse basses, comme par exemple les polymères acryliques, mais ces polymères forment généralement des dépôts très collants ce qui présente un inconvénient dans la plupart des applications cosmétiques.

La demanderesse a découvert de manière surprenante un nouveau groupe de polyuréthannes physiologiquement acceptables formant des films non collants, non cassants et capables de déformations plastique et élastique. Ces propriétés viscoélastiques intéressantes sont dues à la présence, dans le polymère, de longs motifs macromoléculaires ayant une température de transition vitreuse relativement faible et qui de ce fait, à température ambiante, ne se trouvent pas à l'état vitreux.

La présente invention a par conséquent pour objet des polyuréthannes cationiques à caractère élastique, constitués essentiellement

(a1) de motifs cationiques dérivés d'au moins une amine tertiaire présentant au moins deux fonctions réactives à hydrogène labile, ladite amine étant au moins partiellement neutralisée,
(a2) de motifs non ioniques dérivés de polymères non ioniques portant à leurs extrémités des fonctions réactives à hydrogène labile et ayant une température de transition vitreuse ($T_g$), mesurée par analyse enthalpique différentielle, inférieure à 10 °C choisis parmi les copolymères d'éthylène et de butylène, éventuellement
(a3) de motifs non ioniques dérivés de composés non ioniques monomères contenant au moins deux fonctions à hydrogène labile, et
(b) de motifs dérivés d'au moins un diisocyanate.

L'invention a également pour objet l'utilisation des polyuréthannes cationiques à caractère élastique ci-dessus dans des compositions cosmétiques en vue d'améliorer les propriétés visco-élastiques des dépôts et films cosmétiques obtenus à partir de ces compositions.

Elle a en particulier pour objet l'utilisation de ces polyuréthannes dans des laques et des compositions de coiffage, dans des vernis à ongles et dans des compositions de maquillage.

L'invention a également pour objet des compositions cosmétiques contenant les polyuréthannes cationiques à caractère élastique ci-dessus.

L'invention a aussi pour objet des compositions cosmétiques contenant au moins un polyuréthanne cationique à caractère élastique, constitué essentiellement

(a1) de motifs cationiques dérivés d'au moins une amine tertiaire présentant au moins deux fonctions réactives à hydrogène labile, ladite amine étant au moins partiellement neutralisée,
(a2) de motifs non ioniques dérivés de polymères non ioniques portant à leurs extrémités des fonctions réactives à hydrogène labile et ayant une température de transition vitreuse ($T_g$), mesurée par analyse enthalpique différentielle, inférieure à 10 °C, éventuellement
(a3) de motifs non ioniques dérivés de composés non ioniques monomères contenant au moins deux fonctions à hydrogène labile, et
(b) de motifs dérivés d'au moins un diisocyanate,

les motifs (a1) représentant de 5 à 60 % en poids , les motifs (a2) de 40 à 70 % en poids et les motifs (a3) de 0 à 30 % en poids du polymère total, les motifs (b) étant présents en une quantité essentiellement stoechiométrique par rapport à la somme des motifs (a1), (a2) et (a3).

Les polyuréthannes à caractère élastique de la présente invention, grâce à leur charge cationique, présentent l'avantage d'avoir une excellente affinité pour les substrats kératiniques tels que les cheveux, les ongles et la couche

cornée de l'épiderme auxquels la kératine confère une charge négative.

L'utilisation des polyuréthannes cationiques à caractère élastique de la présente invention dans des laques et des compositions de coiffage permet d'améliorer la souplesse de la coiffure, c'est-à-dire d'obtenir une tenue élastique des cheveux plus naturelle que celle obtenue avec des polymères fixants usuels.

On peut utiliser ces polyuréthannes pour couvrir les ongles avec un film protecteur brillant résistant aux agressions mécaniques. Leur incorporation dans des vernis à ongles améliore la résistance aux chocs de ceux-ci et retarde l'écaillage.

Les polyuréthannes cationiques ci-dessus peuvent également être utilisés pour améliorer la tenue de compositions de maquillage de la peau, des lèvres et des phanères. En effet, les produits de maquillage contenant ces polymères adhèrent bien à la peau et aux phanères et les dépôts obtenus suivent les déformations des substrats kératiniques et ne tirent pas la peau.

Dans toutes ces applications, on obtient des produits non collants.

Comme indiqué ci-dessus, les polyuréthannes cationiques à caractère élastique de la présente invention sont constitués essentiellement de trois types de motifs que sont

(a1) des motifs cationiques dérivés d'au moins une amine tertiaire présentant au moins deux fonctions réactives à hydrogène labile, ladite amine tertiaire étant au moins partiellement neutralisée,
(a2) des motifs non ioniques dérivés de polymères non ioniques portant à leurs extrémités des fonctions réactives à hydrogène labile et ayant une température de transition vitreuse (Tg), mesurée par analyse enthalpique différentielle, inférieure à 10 °C, et
(b) des motifs dérivés d'au moins un diisocyanate.

On entend par fonctions réactives à hydrogène labile des fonctions capables, après départ d'un atome d'hydrogène, de former des liaisons covalentes avec les fonctions isocyanate des composés formant les motifs (b). On peut citer à titre d'exemple de telles fonctions les groupes hydroxyle, amine primaire ($-NH_2$) ou amine secondaire (-NHR), ou encore les groupes thiol (-SH).

La polycondensation de composés portant ces fonctions réactives à hydrogène labile avec des diisocyanates donne, selon la nature des fonctions réactives portant l'hydrogène labile (-OH, $-NH_2$, -NHR ou -SH), respectivement des polyuréthannes, des polyurées ou des polythio-uréthannes. Tous ces polymères sont regroupés dans la présente demande, par souci de simplification, sous le terme de polyuréthannes.

Lorsque les amines tertiaires formant les motifs (a1) portent plus de deux fonctions à hydrogène labile, les polyuréthannes obtenus présentent une structure ramifiée.

Dans un mode de réalisation préféré des polyuréthannes de la présente invention, les amines tertiaires formant les motifs cationiques (a1) ne présentent que deux fonctions réactives à hydrogène labile et les polyuréthannes obtenus par polycondensation ont par conséquent une structure essentiellement linéaire.

Il est bien entendu également possible d'utiliser un mélange d'amines difonctionnelles contenant une faible proportion d'amines portant plus de deux fonctions réactives à hydrogène labile.

Les amines tertiaires formant les motifs cationiques (a1) sont de préférence choisies parmi les composés correspondant à l'une des formules suivantes :

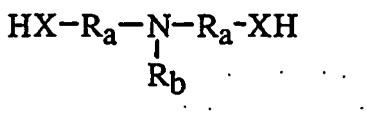

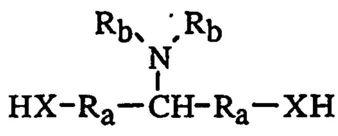

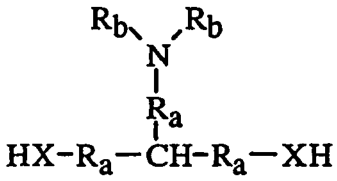

dans lesquelles
chaque $R_a$ représente indépendamment un groupe alkylène en $C_{1-6}$, linéaire ou ramifié, cycloalkylène en $C_{3-6}$ ou arylène, tous pouvant être substitués par un ou plusieurs atomes d'halogène et comporter un ou plusieurs hétéroatomes choisis parmi O, N, P et S,
chaque $R_b$ représente indépendamment un groupe alkyle en $C_{1-6}$, cycloalkyle en $C_{3-6}$ ou aryle, tous pouvant être substitués par un ou plusieurs atomes d'halogène et comporter un ou plusieurs hétéroatomes choisis parmi O, N, P et S, et chaque X représente indépendamment un atome d'oxygène ou de soufre ou un groupe NH ou $NR_c$, où $R_c$ représente un groupe alkyle en $C_{1-6}$.

On peut citer à titre d'amines tertiaires particulièrement préférées pour l'obtention des polyuréthannes cationiques à caractère élastique de la présente invention la N-méthyldiéthanolamine et la N-tert-butyldiéthanolamine.

Les amines tertiaires formant les motifs cationiques (a1) des polyuréthannes de la présente invention peuvent également être des polymères à fonctions amine tertiaire, portant à leurs extrémités des fonctions réactives à hydrogène labile. La masse molaire moyenne en poids de ces polymères à fonctions amine tertiaire est de préférence comprise entre 400 et 10 000.

On peut citer à titre d'exemples de tels polymères à fonctions amine appropriés les polyesters issus de la polycondensation de N-méthyldiéthanolamine et de l'acide adipique.

Comme indiqué ci-dessus, les amines tertiaires formant les motifs cationiques (a1) sont partiellement ou totalement neutralisées par un agent de neutralisation approprié, notamment les acides minéraux ou organiques tels que l'acide chlorhydrique, l'acide bromhydrique, les acides carboxyliques et en particulier les acides monocarboxyliques tels que les acides acétique, propionique, benzoïque, lactique, stéarique et oléique, ou les polyacides. L'acide organique peut éventuellement porter d'autres fonctions telles que OH (acide citrique, acide salicylique).

La neutralisation des fonctions amine tertiaire par un acide approprié est différente de la quaternisation de ces fonctions à l'aide d'un agent de quaternisation et ne doit pas être confondue avec celle-ci.

Le deuxième type de motifs formant les polyuréthannes de la présente invention sont des motifs macromoléculaires, appelés motifs (a2), dérivés de polymères non ioniques portant à leurs extrémités des fonctions réactives à hydrogène labile et ayant une température de transition vitreuse (Tg), mesurée par analyse enthalpique différentielle, inférieure à 10 °C.

Les propriétés viscoélastiques des polyuréthannes sont particulièrement avantageuses lorsque les motifs (a2) sont dérivés de polymères ayant une température de transition vitreuse inférieure à 0 °C et mieux encore inférieure à -10 °C.

Ces polymères ont de préférence une masse molaire moyenne en poids comprise entre 400 et 10 000 et plus particulièrement entre 1000 et 5000.

Les polymères non ioniques susceptibles de former les motifs non ioniques (a2) sont choisis par exemple parmi les polyéthers, les polyesters, les polysiloxanes; les copolymères d'éthylène et de butylène, les polycarbonates et les polymères fluorés.

On préfère tout particulièrement les polyéthers et parmi ceux-ci le poly(tétraméthylène oxyde).

Les diisocyanates formant les motifs (b) englobent les diisocyanates aliphatiques, alicycliques ou aromatiques.

Des diisocyanates préférés sont choisis parmi le méthylènediphényldiisocyanate, le méthylènecyclohexanediisocyanate, l'isophoronediisocyanate, le toluènediisocyanate, le naphtalènediisocyanate, le butanediisocyanate et l'hexyldiisocyanate. Ces diisocyanates peuvent bien entendu être utilisés seuls ou sous forme de mélange de deux ou plusieurs diisocyanates.

Comme indiqué ci-avant, les polyuréthannes cationiques à caractère élastique de la présente invention peuvent contenir, en plus des motifs (a1), (a2) et (b), obligatoirement présents dans les polyuréthannes de la présente invention, une certaine fraction de motifs (a3) dérivés de composés non ioniques monomères contenant au moins deux fonctions à hydrogène labile.

Ces motifs (a3) sont dérivés par exemple de néopentylglycol, d'hexaéthylèneglycol ou d'aminoéthanol.

Le paramètre physique caractérisant les propriétés visco-élastiques des polyuréthannes cationiques ci-dessus est leur recouvrance en traction. Cette recouvrance est déterminée par essai de fluage en traction consistant à étirer rapidement une éprouvette jusqu'à un taux d'allongement prédéterminé, puis à relâcher la contrainte et à mesurer la longueur de l'éprouvette.

L'essai de fluage utilisé pour la caractérisation des polyuréthannes cationiques à caractère élastique dé la présente invention se déroule de la manière suivante :

On utilise, comme éprouvette, un film du polyuréthanne ayant une épaisseur de 500 ± 50 mm, découpé en bandes de 80 mm x 15 mm. Ce film de copolymère est obtenu par séchage, à une température de 22 ± 2 °C et à une humidité relative de 50 ± 5 %, d'une solution ou dispersion à 3 % en poids dudit polyuréthanne dans de l'eau et/ou de l'éthanol.

Chaque bande est fixée entre deux mors, distants de 50 ± 1 mm l'un de l'autre, et est étirée à une vitesse de 20 mm/minute (dans les conditions de température et d'humidité relative ci-dessus) jusqu'à un allongement de 50 % ($\varepsilon_{max}$), c'est-à-dire jusqu'à 1,5 fois sa longueur initiale. On relâche alors la contrainte en imposant une vitesse de retour égale à la vitesse de traction, soit 20 mm/minute, et on mesure l'allongement de l'éprouvette (exprimé en % par rapport à la longueur initiale) immédiatement après retour à charge nulle ($\varepsilon_i$).

La recouvrance instantanée ($R_i$) est calculée à l'aide de la formule suivante :

$$R_i\ (\%) = ((\varepsilon_{max} - \varepsilon_i)/\varepsilon_{max}) \times 100$$

Les polyuréthannes cationiques à caractère élastique de la présente invention ont de préférence une recouvrance instantanée ($R_i$), mesurée dans les conditions indiquées ci-dessus, comprise entre 5 % et 95 %, en particulier comprise entre 20 % et 90 % et idéalement entre 35 et 85 %.

La température de transition vitreuse (Tg) des polymères non ioniques formant les motifs (a2) et des polyuréthannes cationiques de la présente invention est mesurée par analyse enthalpique différentielle (DSC, *differential scanning calorimetry*) selon la norme ASTM D3418-97.

Les polyuréthannes cationiques à caractère élastique de la présente invention présentent de préférence au moins deux températures de transition vitreuse, dont une au moins est inférieure à 10 °C, de préférence inférieure à 0 °C et mieux encore inférieure à -10 °C, et au moins une autre est supérieure ou égale à la température ambiante (20 °C).

La recouvrance instantanée et, par conséquent, les propriétés viscoélastiques des polyuréthannes de la présente invention dépendent des fractions des différents motifs monomères (a1), (a2), (a3) et (b).

La fraction de motifs (a1) doit être suffisante pour conférer aux polymères leur charge positive responsable de leur bonne affinité pour les substrats kératiniques. Les motifs (a2) doivent représenter une fraction en poids suffisante pour que les polyuréthannes présentent au moins une température de transition vitreuse inférieure à 10 °C et ne forment pas des films cassants.

De manière générale, les motifs (a1) représentent de 1 à 90 %, de préférence de 5 à 60 % en poids, les motifs (a2) de 10 à 80 %, de préférence de 40 à 70 % en poids et les motifs (a3) de 0 à 50 % en poids, de préférence de 0 à 30 % en poids du polymères total.

Les motifs (b) sont présents en une quantité essentiellement stoechiométrique par rapport à la somme des motifs (a1), (a2) et (a3). En effet, l'obtention de polyuréthannes ayant des masses molaires importantes suppose un nombre de fonctions isocyanate pratiquement identique au nombre de fonctions à hydrogène labile. L'homme du métier saura choisir un éventuel excès molaire de l'un ou de l'autre type de fonction pour ajuster la masse molaire à la valeur désirée.

Comme indiqué ci avant, les polyuréthannes cationiques à caractère élastique peuvent être incorporés dans de nombreuses compositions cosmétiques dont ils améliorent les propriétés cosmétiques.

La quantité de polyuréthanne présente dans les différentes compositions dépend bien entendu du type de composition et des propriétés recherchées et peut varier à l'intérieur d'une gamme très large, comprise généralement entre 0,5 et 90 % en poids, de préférence entre 1 et 50 % en poids, rapporté à la composition cosmétique finale.

Lorsque les polyuréthannes cationiques à caractère élastique sont incorporés dans des laques pour cheveux leur concentration est généralement comprise entre 0,5 et 15 % en poids. Dans des vernis à ongles ils représentent généralement de0,5 et 40 % en poids de la composition, et des compositions de maquillage de la peau, des lèvres et des phanères contiennent généralement 0,5 à 20 % en poids des polyuréthannes de la présente invention.

On peut également envisager l'utilisation des polyuréthannes cationiques à caractère élastique de la présente invention sous forme pure par exemple pour former un film protecteur sur les ongles.

**Exemple 1**

Synthèse d'un polyuréthanne cationique à caractère élastique

On introduit dans un réacteur thermostaté muni d'un système d'agitation mécanique et d'un réfrigérant les

monomères et solvant suivants :

- 1 mole d'un mélange de monomères de type diol, c'est-à-dire d'un mélange de N-méthyldiéthanolamine et de poly (tétraméthylène oxyde) de masse molaire moyenne en poids égale à 1400, les proportions molaires relatives de ces deux types de monomères étant celles indiquées dans le Tableau 1 ci-dessous, et
- une quantité de méthyléthylcétone telle que la concentration en monomères de type diol soit égale à 75 % en poids.

On chauffe le mélange sous agitation jusqu'à une température de 70 °C, puis on introduit goutte à goutte sous agitation, sur une durée d'environ 2 heures, un faible excès molaire, c'est-à-dire 1,03 moles d'isophoronediisocyanate, Lors de cette addition, on observe une augmentation de la température jusqu'au reflux du solvant.

On prélève à des intervalles réguliers un échantillon dont on trace un spectre d'absorption IR pour suivre la disparition de la bande correspondant aux fonctions isocyanate (2260 $cm^{-1}$).

Lorsque la bande d'absorption des fonctions -NCO ne diminue plus, ce qui est généralement le cas au bout d'environ 5 heures, on laisse refroidir le mélange réactionnel jusqu'à température ambiante, puis on dilue avec de l'acétone jusqu'à une concentration en polymère d'environ 40 % en poids.

On ajoute ensuite au mélange obtenu 20 ml d'éthanol afin de désactiver les fonctions -NCO résiduelles et on poursuit l'agitation à température ambiante jusqu'à disparition totale des fonctions -NCO, c'est-à-dire de la bande d'absorption IR à 2260 $cm^{-1}$.

On ajoute une solution d'acide chlorhydrique (2 mole/l) en une quantité telle que l'on neutralise les groupements amine au taux voulu. On élimine ensuite les différents solvants organiques (méthyléthylcétone, acétone et éthanol) par distillation sous vide à une température de 40 °C.

Après élimination de la phase organique, on ajoute à la solution aqueuse du polymère une quantité d'eau suffisante pour obtenir une concentration de polymère dans l'eau d'environ 25 % en poids.

On prépare de la manière décrite ci-dessus trois polyuréthannes différents (PU1, PU2 et PU3) présentant respectivement un rapport molaire N-méthyldiéthanolamine/poly(tétraméthylène oxyde) égal à 2, 3 et 4.

Le Tableau 1 ci-dessous montre la composition molaire théorique et les caractéristiques physico-chimiques des trois polymères obtenus.

**Tableau 1**

| | Motifs (a1)[1] (moles) | Motifs (a2)[2] (moles) | Motifs (b)[3] (moles) | Indice d'amine théorique | Masse molaire moyenne en poids[4] |
|---|---|---|---|---|---|
| PU1 | 2 | 1 | 3 | 48 | 36 600 |
| PU2 | 3 | 1 | 4 | 63 | 35 400 |
| PU3 | 4 | 1 | 5 | 75 | 16 800 |

**Tableau 1 (suite)**

| | pH de la solution | Extrait sec de la solution aqueuse | Aspect de la solution aqueuse | Température de transition vitreuse[5] |
|---|---|---|---|---|
| PU1 | 3,3 | 23 % en poids | limpide | -78 et +30 °C |
| PU2 | 2,1 | 24 % en poids | limpide | -79 et +48 °C |
| PU3 | 1,7 | 23 % en poids | limpide | -80 et +23 °C |

[1]N-méthyldiéthanolamine

[2]poly(tétraméthylène oxyde) ayant une masse moyenne en poids de 1400, commercialisé sous la dénomination Terathane® 1400 par la société DUPONT..

[3]isophoronediisocanate

[4]mesurée par chromatographie par perméation de gel dans du THF, détection par réfractométrie.

[5] ASTM D3418-97

**Exemple 2**

Préparation de compositions capillaires (laques)

On prépare trois compositions capillaires comprenant chacune, dans un dispositif aérosol, 65 g de diméthyléther et 35 g d'un mélange eau/éthanol (1:2) contenant 3 % en poids d'un des trois polyuréthannes cationiques à caractère élastique synthétisés dans l'Exemple 1.

On obtient des compositions aisément applicables sur les cheveux.

**Exemple 3**

Évaluation des propriétés cosmétiques des compositions capillaires

On applique sur des mèches de cheveux naturels de 5,4 g, 3 g de chacune des compositions de coiffage préparées dans l'Exemple 2, puis on laisse sécher pendant 1 heure.

Un panel de 10 personnes évalue visuellement les propriétés cosmétiques des mèches ainsi traitées en donnant des notes allant de 0 à 50 (une note de 50 correspondant à l'obtention de la propriété cosmétique recherchée)

Les résultats obtenus sont rassemblés dans le Tableau 2.

**Tableau 2**

| | PU1 | PU2 | PU3 |
|---|---|---|---|
| Élasticité | 40 | 40 | 40 |
| Absence de poudrage | 50 | 40 | 45 |
| Brillance | 40 | 40 | 40 |
| Douceur | 30 | 40 | 40 |
| Absence de poissage | 40 | 45 | 40 |
| Qualité de toucher | 20 | 30 | 40 |

**Exemple 4**

Mesure de la recouvrance instantanée

On prépare des films de polyuréthanne à partir de dispersions à 3 % en poids de chacun des polyuréthannes de l'Exemple 1 dans un mélange eau/éthanol (1/2). Le tableau 4 ci-dessous rassemble les valeurs de recouvrance instantanée (exprimées en %) mesurée dans les conditions suivantes :

épaisseur du film : 500 ± 50 mm,
dimension des bandes de 80 mm x 15 mm
conditions de séchage : 22 ± 2 °C, humidité relative de 50 ± 5 %,
distance entre deux mors : 50 ± 1 mm,
vitesse d'étirement = vitesse de retour : 20 mm/minute

La recouvrance instantanée ($R_i$) est calculée à l'aide de la formule suivante :

$$R_i\ (\%) = ((\varepsilon_{max} - \varepsilon_i)/\varepsilon_{max}) \times 100$$

**Tableau 3**

| Polyuréthanne | Recouvrance instantanée |
|---|---|
| PU1 | 40 % |
| PU2 | 63 % |
| PU3 | 48 % |

**Revendications**

**1.** Polyuréthannes cationiques à caractère élastique, **caractérisés par le fait qu'**ils sont constitués essentiellement

(a1) de motifs cationiques dérivés d'au moins une amine tertiaire présentant au moins deux fonctions réactives à hydrogène labile, ladite amine étant au moins partiellement neutralisée,
(a2) de motifs non ioniques dérivés de polymères non ioniques portant à leurs extrémités des fonctions réactives à hydrogène labile et ayant une température de transition vitreuse (Tg), mesurée par analyse enthalpique différentielle, inférieure à 10 °C choisis parmi les copolymères d'éthylène et de butylène, éventuellement
(a3) de motifs non ioniques dérivés de composés non ioniques monomères contenant au moins deux fonctions à hydrogène labile, et

(b) de motifs dérivés d'au moins un diisocyanate.

2. Polyuréthannes cationiques à caractère élastique selon la revendication 1, **caractérisés par le fait que** les polymères non ioniques formant les motifs non ioniques (a2) présentent une température de transition vitreuse, mesurée par analyse enthalpique différentielle, inférieure 0 °C et de préférence inférieure à -10 °C.

3. Polyuréthannes cationiques à caractère élastique selon la revendication 1 ou 2, **caractérisés par le fait qu'**ils présentent au moins deux températures de transitions vitreuses (Tg) différentes, au moins une de ces Tg étant inférieure à 10 °C et au moins une autre étant supérieure ou égale à 20 °C.

4. Polyuréthannes cationiques à caractère élastique selon l'une quelconque des revendications précédentes, **caractérisés par le fait qu'**ils présentent une recouvrance immédiate comprise entre 5 et 95 %, de préférence entre 20 et 90 % et en particulier entre 35 et 85 % en poids.

5. Polyuréthannes cationiques à caractère élastique selon l'une quelconque des revendications précédentes, **caractérisés par le fait que** les motifs cationiques (a1) sont dérivés d'au moins une amine tertiaire, au moins partiellement neutralisée, présentant deux fonctions réactives à hydrogène labile.

6. Polyuréthannes cationiques à caractère élastique selon la revendication 5, **caractérisés par le fait que** les motifs cationiques (a1) sont dérivés d'une amine tertiaire correspondant à l'une des formules suivantes :

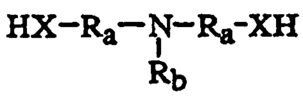

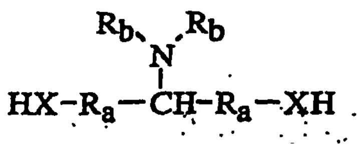

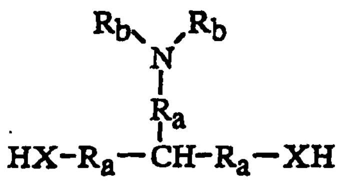

dans lesquelles
chaque $R_a$ représente indépendamment un groupe alkylène en $C_{1-6}$, linéaire ou ramifié, cycloalkylène en $C_{3-6}$ ou arylène, tous pouvant être substitués par un ou plusieurs atomes d'halogène et comporter un ou plusieurs hétéroatomes choisis parmi O, N, P et S;
chaque $R_b$ représente indépendamment un groupe alkyle en $C_{1-6}$, cycloalkyle en $C_{3-6}$ ou aryle, tous pouvant être substitués par un ou plusieurs atomes d'halogène et comporter un ou plusieurs hétéroatomes choisis parmi O, N, P et S, et
chaque X représente indépendamment un atome d'oxygène ou de soufre ou un groupe NH ou $NR_c$, où $R_c$ représente un groupe alkyle en $C_{1-6}$.

7. Polyuréthannes cationiques à caractère élastique selon la revendication 6, **caractérisés par le fait que** les motifs cationiques (a1) sont dérivés de la N-méthyldiéthanolamine et de la N-tert-butyldiéthanolamine.

8. Polyuréthannes cationiques à caractère élastique selon la revendication 5, **caractérisés par le fait que** les motifs

(a1) sont dérivés de polymères à fonctions amine tertiaire, portant à leurs extrémités des fonctions réactives à hydrogène labile choisies parmi -OH, $-NH_2$, $-NR_c$ ou -SH, et ayant une masse moléculaire en poids comprise entre 400 et 10 000, $R_c$ étant défini comme dans la revendication 6.

**9.** Polyuréthannes cationiques à caractère élastique selon l'une quelconque des revendications précédentes, **caractérisés par le fait que** les polymères formant les motifs non ioniques (a2) ont une masse molaire moyenne en poids comprise entre 400 et 10 000, de préférence entre 1000 et 5000.

**10.** Polyuréthannes cationiques à caractère élastique selon l'une quelconque des revendications précédentes, **caractérisés par le fait que** les motifs (b) sont dérivés de diisocyanates choisis parmi le méthylène diphényldiisocyanate, le méthylènecyclohexanediisocyanate, l'isophoronediisocyanate, le toluènediisocyanate, le naphtalènediisocyanate, le butanediisocyanate et l'hexyldiisocyanate.

**11.** Polyuréthannes cationiques à caractère élastique selon l'une quelconque des revendications précédentes, **caractérisés par le fait que** les composés non ioniques formant les motifs non ioniques monomères (a3), éventuellement présents, sont choisis parmi le néopentylglycol, l'hexaéthylèneglycol et l'aminoéthanol.

**12.** Polyuréthannes cationiques à caractère élastique selon l'une quelconque des revendications précédentes, **caractérisés par le fait que** les motifs (a1) représentent de 1 à 90 %, de préférence de 5 à 60 % en poids, les motifs (a2) de 10 à 80 %, de préférence de 40 à 70 % en poids et les motifs (a3) de 0 à 50 % en poids, de préférence de 0 à 30 % en poids du polymères total, les motifs (b) étant présents en une quantité essentiellement stoechiométrique par rapport à la somme des motifs (a1), (a2) et (a3).

**13.** Composition cosmétique contenant, au moins un polyuréthanne cationique à caractère élastique, constitué essentiellement

(a1) de motifs cationiques dérivés d'au moins une amine tertiaire présentant au moins deux fonctions réactives à hydrogène labile, ladite amine étant au moins partiellement neutralisée,
(a2) de motifs non ioniques dérivés de polymères non ioniques portant à leurs extrémités des fonctions réactives à hydrogène labile et ayant une température de transition vitreuse (Tg), mesurée par analyse enthalpique différentielle, inférieure à 10 °C, éventuellement
(a3) de motifs non ioniques dérivés de composés non ioniques monomères contenant au moins deux fonctions à hydrogène labile, et
(b) de motifs dérivés d'au moins un diisocyanate

les motifs (a1) représentant de 5 à 60 % en poids, les motifs (a2) de 40 à 70 % en poids et les motifs (a3) de 0 à 30 % en poids du polymères total, les motifs (b) étant présents en une quantité essentiellement stoechiométrique par rapport à la somme des motifs (a1), (a2) et (a3).

**14.** Composition cosmétique selon la revendication 13 **caractérisée par le fait que** les polymères non ioniques formant les motifs non ioniques (a2) présentent une température de transition vitreuse, mesurée par analyse enthalpique différentielle, inférieure 0 °C et de préférence inférieure à -10 °C.

**15.** Composition cosmétique selon la revendication 13 ou 14, **caractérisée par le fait que** les polyuréthannes cationiques à caractère élastique présentent au moins deux températures de transitions vitreuses (Tg) différentes, au moins une de ces Tg étant inférieure à 10 °C et au moins une antre étant supérieure ou égale à 20 °C.

**16.** Composition cosmétique selon l'une quelconque des revendications 13 à 15, **caractérisée par le fait que** les polyuréthannes cationiques à caractère élastique présentent une recouvrance immédiate comprise entre 5 et 95 %, de préférence entre 20 et 90 % et en particulier entre 35 et 85 % en poids.

**17.** Composition cosmétique selon l'une quelconque des revendications 13 à 16, **caractérisée par le fait que** les motifs cationiques (a1) sont dérivés d'au moins une amine tertiaire, au moins partiellement neutralisée, présentant deux fonctions réactives à hydrogène labile.

**18.** Composition cosmétique selon la revendication 17, **caractérisée par le fait que** les motifs cationiques (a1) sont dérivés d'une amine tertiaire correspondant à l'une des formules suivantes :

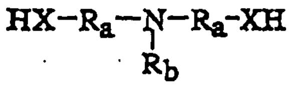

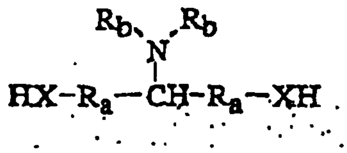

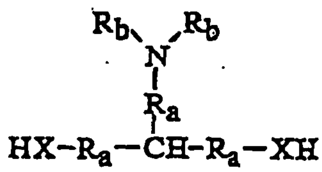

dans lesquelles
chaque $R_a$ représente indépendamment un groupe alkylène en $C_{1-6}$, linéaire ou ramifié, cycloalkylène en $C_{3-6}$ on arylène, tous pouvant être substitués par un ou plusieurs atomes d'halogène et comporter un ou plusieurs hétéroatomes choisis parmi O, N, P et S,
chaque $R_b$ représente indépendamment un groupe alkyle en $C_{1-6}$, cycloalkyle en $C_{3-6}$ on aryle, tous pouvant être substitués par un ou plusieurs atomes d'halogène et comporter un on plusieurs hétéroatomes choisis parmi O, N, P et S, et
chaque X représente indépendamment un atome d'oxygène ou de soufre ou un groupe NH ou $NR_c$, où $R_c$ représente un groupe alkyle en $C_{1-6}$.

**19.** Composition cosmétique selon la revendication 18, **caractérisée par le fait que** les motifs cationiques (a1) sont dérivés de la N-méthyldiéthanolamine et de la N-tert-butyldiéthanolamine.

**20.** Composition cosmétique selon la revendication 17, **caractérisée par le fait que** les motifs (a1) sont dérivés de polymères à fonctions amine tertiaire, portant à leurs extrémités des fonctions réactives à hydrogène labile choisies parmi -OH, $-NH_2$, $-NR_c$ ou -SH, et ayant une masse moléculaire en poids comprise entre 400 et 10 000, $R_c$ étant défini comme dans la revendication 18.

**21.** Composition cosmétique selon l'une quelconque des revendications 13 à 20, **caractérisée par le fait que** les polymères formant les motifs non ioniques (a2) sont choisis parmi les polyéthers, les polyesters, les polysiloxanes, les copolymères d'éthylène et de butylène, les polycarbonates ou les polymères fluorés.

**22.** Composition cosmétique selon l'une quelconque des revendications 13 à 21, **caractérisée par le fait que** les polymères formant les motifs non ioniques (a2) ont une masse molaire moyenne en poids comprise entre 400 et 10 000, de préférence entre 1000 et 5000.

**23.** Composition cosmétique selon la revendication 13 à 22, **caractérisée par le fait que** les motifs non ioniques (a2) sont dérivés de poly(tétraméthylène oxyde).

**24.** Composition cosmétique selon l'une quelconque des revendications 13 à 23, **caractérisée par le fait que** les motifs (b) sont dérivés de diisocyanates choisis parmi le méthylènediphényldiisocyanate, le méthylènecycloboxanediisocyanate, l'isophoronediisocyanate, le toluènediisocyanate, le naphtalènediisocyanate, le butanediisocyanate et l'hexyldiisocyanate.

**25.** Composition cosmétique selon l'une quelconque des revendications 13 à 24, **caractérisée par le fait que** les composés non ioniques formant les motifs non ioniques monomères (a3), éventuellement présents, sont choisis parmi le néopentylglycol, l'hexaéthylèneglycol et l'aminoéthanol.

**26.** Composition cosmétique selon l'une quelconque des revendications 13 à 25, **caractérisée par le fait qu'**il s'agit d'une laque pour cheveux et qu'elle contient de 0,5 à 15 % en poids de polyuréthanne cationique tel que défini à l'une quelconque des revendications 13 à 25.

**27.** Composition cosmétique selon l'une quelconque des revendications 13 à 25, **caractérisée par le fait qu'**il s'agit d'un vernis à ongles et qu'elle contient de 0,5 à 40 % en poids de polyuréthanne cationique tel que défini à l'une quelconque des revendications 13 à 25.

**28.** Composition cosmétique selon l'une quelconque des revendications 13 à 25, **caractérisée par le fait qu'**il s'agit d'une composition de maquillage de la peau, des lèvres et des phanères et qu'elle contient de 0,5 à 20 % en poids de polyuréthanne cationique tel que défini à l'une quelconque des revendications 13 à 25.

**29.** Utilisation des polyuréthannes cationiques à caractère élastique selon l'une des revendications 1 à 12 dans une laque de cheveux.

**30.** Utilisation des polyuréthannes cationiques à caractère élastique selon l'une des revendications 1 à 12 dans un vernis à ongles.

**31.** Utilisation des polyuréthannes cationiques à caractère élastique selon l'une des revendications 1 à 12 pour former un film protecteur sur les ongles.

**32.** Utilisation des polyuréthannes cationiques à caractère élastique selon l'une des revendications 1 à 12 dans une composition de maquillage de la peau, des lèvres et des phanères.

## Claims

**1.** Cationic polyurethanes with an elastic nature, **characterized in that** they are composed essentially:

(a1) of cationic units derived from at least one tertiary amine exhibiting at least two reactive functional groups comprising labile hydrogen, said amine being at least partially neutralized,
(a2) of nonionic units derived from nonionic polymers carrying, at their ends, reactive functional groups comprising labile hydrogen and having a glass transition temperature (Tg), measured by differential scanning calorimetry, of less than 10°C chosen from ethylene/butylene copolymers, optionally
(a3) of nonionic units derived from monomeric nonionic compounds comprising at least two functional groups comprising labile hydrogen, and
(b) of units derived from at least one diisocyanate.

**2.** Cationic polyurethanes with an elastic nature according to Claim 1, **characterized in that** the nonionic polymers forming the nonionic units (a2) exhibit a glass transition temperature, measured by differential scanning calorimetry, of less than 0°C and preferably of less than -10°C.

**3.** Cationic polyurethanes with an elastic nature according to Claim 1 or 2, **characterized in that** they exhibit at least two different glass transition temperatures (Tg), at least one of these Tg values being less than 10°C and at least one other being greater than or equal to 20°C.

**4.** Cationic polyurethanes with an elastic nature according to any one of the preceding claims, **characterized in that** they exhibit an immediate recovery of between 5 and 95% by weight, preferably between 20 and 90% by weight and in particular between 35 and 85% by weight.

**5.** Cationic polyurethanes with an elastic nature according to any one of the preceding claims, **characterized in that** the cationic units (a1) are derived from at least one at least partially neutralized tertiary amine exhibiting two reactive functional groups comprising labile hydrogen.

**6.** Cationic polyurethanes with an elastic nature according to Claim 5, **characterized in that** the cationic units (a1) are derived from a tertiary amine corresponding to one of the following formulae:

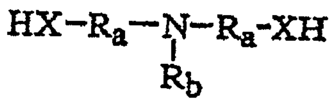

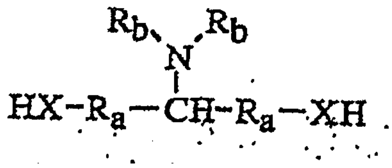

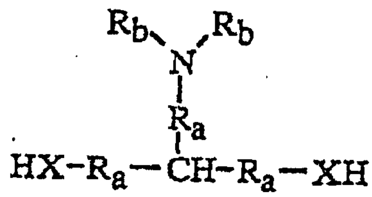

in which:

each $R_a$ independently represents a linear or branched $C_{1-6}$ alkylene group, a $C_{3-6}$ cycloalkylene group or an arylene group, it being possible for all to be substituted by one or more halogen atoms and to comprise one or more heteroatoms chosen from O, N, P and S,

each $R_b$ independently represents a $C_{1-6}$ alkyl, $C_{3-6}$ cycloalkyl or aryl group, it being possible for all to be substituted by one or more halogen atoms and to comprise one or more heteroatoms chosen from O, N, P and S, and

each X independently represents an oxygen or sulphur atom or an NH or $NR_c$ group where $R_c$ represents a $C_{1-6}$ alkyl group.

**7.** Cationic polyurethanes with an elastic nature according to Claim 6, **characterized in that** the cationic units (a1) are derived from N-methyldiethanolamine and N-(tert-butyl)diethanolamine.

**8.** Cationic polyurethanes with an elastic nature according to Claim 5, **characterized in that** the units (a1) are derived from polymers comprising tertiary amine functional groups carrying, at their ends, reactive functional groups comprising labile hydrogen chosen from -OH, $-NH_2$, $-NR_c$ or -SH and having a weight-average molecular weight of between 400 and 10,000, $R_c$ being defined as in Claim 6.

**9.** Cationic polyurethanes with an elastic nature according to any one of the preceding claims, **characterized in that** the polymers forming the nonionic units (a2) have a weight-average molar mass of between 400 and 10,000, preferably between 1000 and 5000.

**10.** Cationic polyurethanes with an elastic nature according to any one of the preceding claims, **characterized in that** the units (b) are derived from diisocyanates chosen from methylenediphenyl diisocyanate, methylenecyclohexane diisocyanate, isophorone diisocyanate, toluene diisocyanate, naphthalene diisocyanate, butane diisocyanate and hexyl diisocyanate.

**11.** Cationic polyurethanes with an elastic nature according to any one of the preceding claims, **characterized in that** the nonionic compounds forming the monomeric nonionic units (a3) optionally present are chosen from neopentyl glycol, hexaethylene glycol and aminoethanol.

**12.** Cationic polyurethanes with an elastic nature according to any one of the preceding claims, **characterized in that** the units (a1) represent from 1 to 90% by weight, preferably from 5 to 60% by weight, the units (a2) from 10 to 80% by weight, preferably from 40 to 70% by weight, and the units (a3) from 0 to 50% by weight, preferably from 0 to

30% by weight, of the total polymer, the units (b) being present in an essentially stoichiometric amount with respect to the sum of the units (a1), (a2) and (a3).

**13.** Cosmetic composition comprising at least one cationic polyurethane with an elastic nature composed essentially

(a1) of cationic units derived from at least one tertiary amine exhibiting at least two reactive functional groups comprising labile hydrogen, said amine being at least partially neutralized,
(a2) of nonionic units derived from nonionic polymers carrying, at their ends, reactive functional groups comprising labile hydrogen and having a glass transition temperature (Tg), measured by differential scanning calorimetry, of less than 10°C, optionally
(a3) of nonionic units derived from monomeric nonionic compounds comprising at least two functional groups comprising labile hydrogen, and
(b) of units derived from at least one diisocyanate,

the units (a1) representing from 5 to 60% by weight, the units (a2) from 40 to 70% by weight and the units (a3) from 0 to 30% by weight of the total polymer, the units (b) being present in an essentially stoichiometric amount with respect to the sum of the units (a1), (a2) and (a3).

**14.** Cosmetic composition according to Claim 13, **characterized in that** the nonionic polymers forming the nonionic units (a2) exhibit a glass transition temperature, measured by differential scanning calorimetry, of less than 0°C and preferably of less than -10°C.

**15.** Cosmetic composition according to Claim 13 or 14, **characterized in that** the cationic polyurethanes with an elastic nature exhibit at least two different glass transition temperatures (Tg), at least one of these Tg values being less than 10°C and at least one other being greater than or equal to 20°C.

**16.** Cosmetic composition according to any one of Claims 13 to 15, **characterized in that** the cationic polyurethanes with an elastic nature exhibit an immediate recovery of between 5 and 95% by weight, preferably between 20 and 90% by weight and in particular between 35 and 85% by weight.

**17.** Cosmetic composition according to any one of Claims 13 to 16, **characterized in that** the cationic units (a1) are derived from at least one at least partially neutralized tertiary amine exhibiting two reactive functional groups comprising labile hydrogen.

**18.** Cosmetic composition according to Claim 17, **characterized in that** the cationic units (a1) are derived from a tertiary amine corresponding to one of the following formulae:

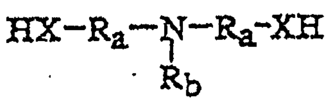

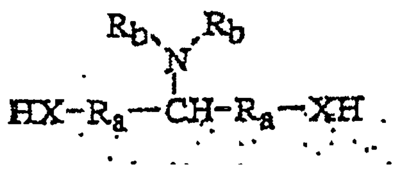

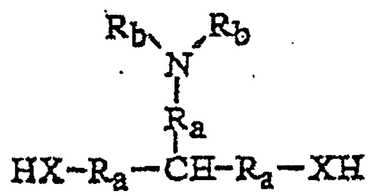

in which:

each $R_a$ independently represents a linear or branched $C_{1-6}$ alkylene group, a $C_{3-6}$ cycloalkylene group or an arylene group, it being possible for all to be substituted by one or more halogen atoms and to comprise one or more heteroatoms chosen from O, N, P and S,
each $R_b$ independently represents a $C_{1-6}$ alkyl, $C_{3-6}$ cycloalkyl or aryl group, it being possible for all to be substituted by one or more halogen atoms and to comprise one or more heteroatoms chosen from O, N, P and S, and
each X independently represents an oxygen or sulphur atom or an NH or $NR_c$ group where $R_c$ represents a $C_{1-6}$ alkyl group.

**19.** Cosmetic composition according to Claim 18, **characterized in that** the cationic units (a1) are derived from N-methyldiethanolamine and N-(tert-butyl)diethanolamine.

**20.** Cosmetic composition according to Claim 17, **characterized in that** the units (a1) are derived from polymers comprising tertiary amine functional groups carrying, at their ends, reactive functional groups comprising labile hydrogen chosen from -OH, $-NH_2$, $-NR_c$ or -SH and having a weight-average molecular weight of between 400 and 10,000, $R_c$ being defined as in Claim 18.

**21.** Cosmetic composition according to any one of Claims 13 to 20, **characterized in that** the polymers forming the nonionic units (a2) are chosen from polyethers, polyesters, polysiloxanes, ethylene/butylene copolymers, polycarbonates or fluoropolymers.

**22.** Cosmetic composition according to any one of Claims 13 to 21, **characterized in that** the polymers forming the nonionic units (a2) have a weight-average molar mass of between 400 and 10,000, preferably between 1000 and 5000.

**23.** Cosmetic composition according to Claim 13 to 22, **characterized in that** the nonionic units (a2) are derived from poly(tetramethylene oxide).

**24.** Cosmetic composition according to any one of Claims 13 to 23, **characterized in that** the units (b) are derived from diisocyanates chosen from methylenediphenyl diisocyanate, methylenecyclohexane diisocyanate, isophorone diisocyanate, toluene diisocyanate, naphthalene diisocyanate, butane diisocyanate and hexyl diisocyanate.

**25.** Cosmetic composition according to any one of Claims 13 to 24, **characterized in that** the nonionic compounds forming the monomeric nonionic units (a3) optionally present are chosen from neopentyl glycol, hexaethylene glycol and aminoethanol.

**26.** Cosmetic composition according to any one of Claims 13 to 25, **characterized in that** it is a hair lacquer and **in that** it comprises from 0.5 to 15% by weight of cationic polyurethane as defined in any one of Claims 13 to 25.

**27.** Cosmetic composition according to any one of Claims 13 to 25, **characterized in that** it is a nail varnish and that it comprises from 0.5 to 40% by weight of cationic polyurethane as defined in any one of Claims 13 to 25.

**28.** Cosmetic composition according to any one of Claims 13 to 25, **characterized in that** it is a composition for making up the skin, lips and superficial body growths and that it comprises from 0.5 to 20% by weight of cationic polyurethane as defined in any one of Claims 13 to 25.

**29.** Use of the cationic polyurethanes with an elastic nature according to one of Claims 1 to 12 in a hair lacquer.

**30.** Use of the cationic polyurethanes with an elastic nature according to one of Claims 1 to 12 in a nail varnish.

**31.** Use of the cationic polyurethanes with an elastic nature according to one of Claims 1 to 12 for forming a protective film on the nails.

**32.** Use of the cationic polyurethanes with an elastic nature according to one of Claims 1 to 12 in a composition for making up the skin, lips and superficial body growths.

## Patentansprüche

**1.** Kationische Polyurethane mit elastischem Charakter, **dadurch gekennzeichnet, dass** sie im Wesentlichen aus

(a1) kationischen Einheiten, die von mindestens einem tertiären Amin abgeleitet sind, das mindestens zwei reaktive funktionelle Gruppen mit labilem Wasserstoff aufweist, wobei das Amin mindestens teilweise neutralisiert ist,
(a2) nichtionischen Einheiten, die von nichtionischen Polymeren abgeleitet sind, die an ihren Enden reaktive funktionelle Gruppen mit labilem Wasserstoff tragen und die eine Glasübergangstemperatur (Tg), die durch Differentialthermoanalyse gemessen wird, unterhalb von 10 °C aufweisen, die unter den Copolymeren von Ethylen und Butylen ausgewählt werden, gegebenenfalls
(a3) nichtionischen Einheiten, die von monomeren nichtionischen Verbindungen abgeleitet sind, die mindestens zwei funktionelle Gruppen mit labilem Wasserstoff enthalten, und
(b) Einheiten, die von mindestens einem Diisocyanat abgeleitet sind,

bestehen.

**2.** Kationische Polyurethane mit elastischem Charakter nach Anspruch 1, **dadurch gekennzeichnet, dass** die nichtionischen Polymere, die die nichtionischen Einheiten (a2) bilden, eine Glasübergangstemperatur aufweisen, die durch Differentialthermoanalyse gemessen wird, die unter 0 °C und vorzugsweise unter -10 °C liegt.

**3.** Kationische Polyurethane mit elastischem Charakter nach Anspruch 1 oder 2, **dadurch gekennzeichnet, dass** sie mindestens zwei verschiedene Glasübergangstemperaturen (Tg) aufweisen, wobei mindestens eine dieser Glasübergangstemperaturen (Tg) unter 10 °C liegt und mindestens eine andere Glasübergangstemperatur (Tg) bei 20 °C oder darüber liegt.

**4.** Kationische Polyurethane mit elastischem Charakter nach einem der vorhergehenden Ansprüche, **dadurch gekennzeichnet, dass** sie eine unmittelbare Erholung aufweisen, die im Bereich von 5 bis 95 %, vorzugsweise im Bereich von 20 bis 90 % und vor allem im Bereich von 35 bis 85 %, bezogen auf das Gewicht, liegt.

**5.** Kationische Polyurethane mit elastischem Charakter nach einem der vorhergehenden Ansprüche, **dadurch gekennzeichnet, dass** die kationischen Einheiten (a1) von mindestens einem tertiären Amin, das mindestens teilweise neutralisiert ist, abgeleitet sind, das zwei reaktive funktionelle Gruppen mit labilem Wasserstoff aufweist.

**6.** Kationische Polyurethane mit elastischem Charakter nach Anspruch 5, **dadurch gekennzeichnet, dass** die kationischen Einheiten (a1) von einem tertiären Amin abgeleitet sind, das einer der folgenden Formeln

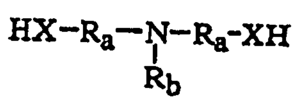

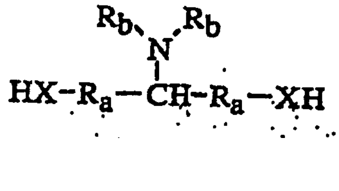

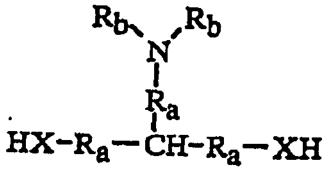

entspricht, in denen bedeuten:

- jedes $R_a$ unabhängig eine geradkettige oder verzweigte $C_{1-6}$-Alkylengruppe, $C_{3-6}$-Cycloalkylengruppe oder Arylengruppe, die alle mit einem oder mehreren Halogenatomen substituiert sein können und ein oder mehrere Heteroatome umfassen können, die unter 0, N, P und S ausgewählt werden;
- jedes $R_b$ unabhängig eine $C_{1-6}$-Alkylgruppe, $C_{3-6}$-Cycloalkylgruppe oder Arylgruppe, die alle mit einem oder mehreren Halogenatomen substituiert sein können und ein oder mehrere Heteroatome umfassen können, die unter O, N, P und S ausgewählt werden, und
- jedes X unabhängig ein Sauerstoffatom oder Schwefelatom oder eine Gruppe NH oder $NR_C$, worin $R_C$ eine $C_{1-6}$-Alkylgruppe darstellt.

**7.** Kationische Polyurethane mit elastischem Charakter nach Anspruch 6, **dadurch gekennzeichnet, dass** die kationischen Einheiten (a1) von N-Methyldiethanolamin und N-tert.-Butyldiethanolamin abgeleitet sind.

**8.** Kationische Polyurethane mit elastischem Charakter nach Anspruch 5, **dadurch gekennzeichnet, dass** die Einheiten (a1) von Polymeren mit tertiären Amingruppen abgeleitet sind, die an ihren Enden reaktive funktionelle Gruppen mit labilem Wasserstoff tragen, die unter -OH, $-NH_2$, $-NR_C$ oder -SH ausgewählt werden und die eine gewichtsmittlere Molekularmasse aufweisen, die im Bereich von 400 bis 10000 liegt, wobei $R_C$ wie in Anspruch 6 definiert ist.

**9.** Kationische Polyurethane mit elastischem Charakter nach einem der vorhergehenden Ansprüche, **dadurch gekennzeichnet, dass** die Polymere, die die nichtionischen Einheiten (a2) bilden, eine gewichtsmittlere Molmasse aufweisen, die im Bereich von 400 bis 10000 und vorzugsweise im Bereich von 1000 bis 5000 liegt.

**10.** Kationische Polyurethane mit elastischem Charakter nach einem der vorhergehenden Ansprüche, **dadurch gekennzeichnet, dass** die Einheiten (b) von Diisocyanaten abgeleitet sind, die unter Methylendiphenyldiisocyanat, Methylencyclohexandiisocyanat, Isophorondiisocyanat, Toluoldiisocyanat, Naphthalindiisocyanat, Butandiisocyanat und Hexyldiisocyanat ausgewählt werden.

**11.** Kationische Polyurethane mit elastischem Charakter nach einem der vorhergehenden Ansprüche, **dadurch gekennzeichnet, dass** die nichtionischen Verbindungen, die die gegebenenfalls vorhandenen monomeren nichtionischen Einheiten (a3) bilden, unter Neopentylglycol, Hexaethylenglycol und Aminoethanol ausgewählt werden.

**12.** Kationische Polyurethane mit elastischem Charakter nach einem der vorhergehenden Ansprüche, **dadurch gekennzeichnet, dass** die Einheiten (a1) 1 bis 90 Gew.-% und vorzugsweise 5 bis 60 Gew.-%, die Einheiten (a2) 10 bis 80 Gew.-% und vorzugsweise 40 bis 70 Gew.-% und die Einheiten (a3) 0 bis 50 Gew.-% und vorzugsweise 0 bis 30 Gew.-% des Gesamtpolymers ausmachen, wobei die Einheiten (b) in einer Menge vorhanden sind, die im Wesentlichen stöchiometrisch ist, bezogen auf die Summe der Einheiten (a1), (a2) und (a3).

**13.** Kosmetische Zusammensetzung, die mindestens ein kationisches Polyurethan mit elastischem Charakter enthält, das im Wesentliche aus

(a1) kationischen Einheiten, die von mindestens einem tertiären Amin abgeleitet sind, das mindestens zwei reaktive funktionelle Gruppen mit labilem Wasserstoff aufweist, wobei das Amin mindestens teilweise neutralisiert ist,
(a2) nichtionischen Einheiten, die von nichtionischen Polymeren abgeleitet sind, die an ihren Enden reaktive funktionelle Gruppen mit labilem Wasserstoff tragen und die eine Glasübergangstemperatur (Tg), die durch Differentialthermoanalyse gemessen wird, unterhalb von 10 °C aufweisen, gegebenenfalls

(a3) nichtionischen Einheiten, die von monomeren nichtionischen Verbindungen abgeleitet sind, die mindestens zwei funktionelle Gruppen mit labilem Wasserstoff enthalten, und
(b) Einheiten, die von mindestens einem Diisocyanat abgeleitet sind,

besteht, wobei die Einheiten (a1) 5 bis 60 Gew.-%, die Einheiten (a2) 40 bis 70 Gew.-% und die Einheiten (a3) 0 bis 30 Gew.-% des Gesamtpolymers ausmachen, wobei die Einheiten (b) in einer Menge vorhanden sind, die im Wesentlichen stöchiometrisch ist, bezogen auf die Summe der Einheiten (a1), (a2) und (a3).

**14.** Kosmetische Zusammensetzung nach Anspruch 13, **dadurch gekennzeichnet, dass** die nichtionischen Polymere, die die nichtionischen Einheiten (a2) bilden, eine Glasübergangstemperatur, die durch Differentialthermoanalyse gemessen wird, aufweisen, die unter 0 °C und vorzugsweise unter -10 °C liegt.

**15.** Kosmetische Zusammensetzung nach Anspruch 13 oder 14, **dadurch gekennzeichnet, dass** die kationischen Polyurethane mit elastischem Charakter mindestens zwei verschiedene Glasübergangstemperaturen (Tg) aufweisen, wobei mindestens eine dieser Glasübergangstemperaturen (Tg) unter 10 °C liegt und mindestens eine andere Glasübergangstemperatur (Tg) bei 20 °C oder darüber liegt.

**16.** Kosmetische Zusammensetzung nach einem der Ansprüche 13 bis 15, **dadurch gekennzeichnet, dass** die kationischen Polyurethane mit elastischem Charakter eine unmittelbare Erholung aufweisen, die im Bereich von 5 bis 95 %, vorzugsweise im Bereich von 20 bis 90 % und vor allem im Bereich von 35 bis 85 %, bezogen auf das Gewicht, liegt.

**17.** Kosmetische Zusammensetzung nach einem der Ansprüche 13 bis 16, **dadurch gekennzeichnet, dass** die kationischen Einheiten (a1) von mindestens einem tertiären Amin, das mindestens teilweise neutralisiert ist, abgeleitet sind, das zwei reaktive funktionelle Gruppen mit labilem Wasserstoff aufweist.

**18.** Kosmetische Zusammensetzung nach Anspruch 17, **dadurch gekennzeichnet, dass** die kationischen Einheiten (a1) von einem tertiären Amin abgeleitet sind, das einer der folgenden Formeln

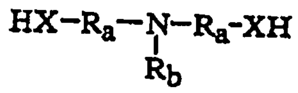

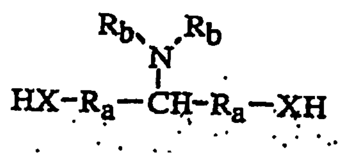

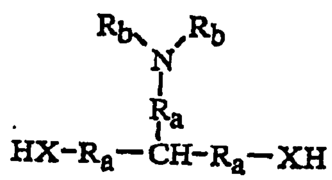

entspricht, in denen bedeuten:

- jedes $R_a$ unabhängig eine geradkettige oder verzweigte $C_{1-6}$-Alkylengruppe, $C_{3-6}$-Cycloalkylengruppe oder Arylengruppe, die alle mit einem oder mehreren Halogenatomen substituiert sein können und ein oder mehrere Heteroatome umfassen können, die unter 0, N, P und S ausgewählt werden;
- jedes $R_b$ unabhängig eine $C_{1-6}$-Alkylgruppe, $C_{3-6}$-Cycloalkylgruppe oder Arylgruppe, die alle mit einem oder mehreren Halogenatomen substituiert sein können und ein oder mehrere Heteroatome umfassen können, die unter O, N, P und S ausgewählt werden, und

- jedes X unabhängig ein Sauerstoffatom oder Schwefelatom oder eine Gruppe NH oder $NR_C$, worin $R_C$ eine $C_{1-6}$-Alkylgruppe darstellt.

**19.** Kosmetische Zusammensetzung nach Anspruch 18, **dadurch gekennzeichnet, dass** die kationischen Einheiten (a1) von N-Methyldiethanolamin und N-tert.-Butyldiethanolamin abgeleitet sind.

**20.** Kosmetische Zusammensetzung nach Anspruch 17, **dadurch gekennzeichnet, dass** die Einheiten (a1) von Polymeren mit tertiären Amingruppen abgeleitet sind, die an ihren Enden reaktive funktionelle Gruppen mit labilem Wasserstoff tragen, die unter -OH, $-NH_2$, $-NR_C$ oder -SH ausgewählt werden und die eine gewichtsmittlere Molekularmasse aufweisen, die im Bereich von 400 bis 10000 liegt, wobei $R_C$ wie in Anspruch 18 definiert ist.

**21.** Kosmetische Zusammensetzung nach einem der Ansprüche 13 bis 20, **dadurch gekennzeichnet, dass** die Polymere, die die nichtionischen Einheiten (a2) bilden, unter den Polyethern, den Polyestern, den Polysiloxanen, den Copolymeren von Ethylen und Butylen, den Polycarbonaten und den fluorierten Polymeren ausgewählt werden.

**22.** Kosmetische Zusammensetzung nach einem der Ansprüche 13 bis 21, **dadurch gekennzeichnet, dass** die Polymere, die die nichtionischen Einheiten (a2) bilden, eine gewichtsmittlere Molmasse aufweisen, die im Bereich von 400 bis 10000 und vorzugsweise im Bereich von 1000 bis 5000 liegt.

**23.** Kosmetische Zusammensetzung nach Anspruch 13 bis 22, **dadurch gekennzeichnet, dass** die nichtionischen Einheiten (a2) von Poly(tetramethylenoxid) abgeleitet sind.

**24.** Kosmetische Zusammensetzung nach einem der Ansprüche 13 bis 23, **dadurch gekennzeichnet, dass** die Einheiten (b) von Diisocyanaten abgeleitet sind, die unter Methylendiphenyldiisocyanat, Methylencyclohexandiisocyanat, Isophorondiisocyanat, Toluoldiisocyanat, Naphthalindiisocyanat, Butandiisocyanat und Hexyldiisocyanat ausgewählt werden.

**25.** Kosmetische Zusammensetzung nach einem der Ansprüche 13 bis 24, **dadurch gekennzeichnet, dass** die nichtionischen Verbindungen, die die gegebenenfalls vorhandenen monomeren nichtionischen Einheiten (a3) bilden, unter Neopentylglycol, Hexaethylenglycol und Aminoethanol ausgewählt werden.

**26.** Kosmetische Zusammensetzung nach einem der Ansprüche 13 bis 25, **dadurch gekennzeichnet, dass** es sich um einen Haarlack handelt und dass sie 0,5 bis 15 Gew.-% des kationischen Polyurethans enthält, das wie in einem der Ansprüche 13 bis 25 definiert ist.

**27.** Kosmetische Zusammensetzung nach einem der Ansprüche 13 bis 25, **dadurch gekennzeichnet, dass** es sich um einen Nagellack handelt und dass sie 0,5 bis 40 Gew.-% des kationischen Polyurethans enthält, das wie in einem der Ansprüche 13 bis 25 definiert ist.

**28.** Kosmetische Zusammensetzung nach einem der Ansprüche 13 bis 25, **dadurch gekennzeichnet, dass** es sich um eine Zusammensetzung zum Schminken der Haut, der Lippen und der Hautanhangsgebilde handelt und dass sie 0,5 bis 20 Gew.-% des kationischen Polyurethans enthält, das wie in einem der Ansprüche 13 bis 25 definiert ist.

**29.** Verwendung der kationischen Polyurethane mit elastischem Charakter nach einem der Ansprüche 1 bis 12 in einem Haarlack.

**30.** Verwendung der kationischen Polyurethane mit elastischem Charakter nach einem der Ansprüche 1 bis 12 in einem Nagellack.

**31.** Verwendung der kationischen Polyurethane mit elastischem Charakter nach einem der Ansprüche 1 bis 12 für die Erzeugung eines Schutzfilms auf den Nägeln.

**32.** Verwendung der kationischen Polyurethane mit elastischem Charakter nach einem der Ansprüche 1 bis 12 in einer Zusammensetzung zum Schminken der Haut, der Lippen und der Hautanhangsgebilde.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9413724 A
- DE 4241118
- EP 0619111 A